Europäisches Patentamt

European Patent Office

Office européen des brevets

(11) Numéro de publication: **0 263 012**
**A1**

(12) **DEMANDE DE BREVET EUROPEEN**

(21) Numéro de dépôt: **87402062.1**

(22) Date de dépôt: **15.09.87**

(51) Int. Cl.⁴: **A 61 F 2/14**

(30) Priorité: **15.09.86 FR 8612880**

(43) Date de publication de la demande:
**06.04.88 Bulletin 88/14**

(84) Etats contractants désignés:
**BE CH DE ES FR GB GR IT LI LU NL**

(71) Demandeur: **Vachet, Jean-Marc**
**15, rue de Buci**
**F-75006 Paris (FR)**

(72) Inventeur: **Vachet, Jean-Marc**
**15, rue de Buci**
**F-75006 Paris (FR)**

(74) Mandataire: **Ahner, Francis et al**
**CABINET REGIMBEAU 26, avenue Kléber**
**F-75116 Paris (FR)**

(54) **Procédé de réalisation d'un implant intra-orbitaire et implant intra-orbitaire.**

(57) La présente invention concerne un procédé de réalisation d'un implant intra-orbitaire, ainsi qu'un implant intra-orbitaire.

Cet implant (1) est réalisé par revêtement intégral d'un noyau sphérique (36), en matériau synthétique bio-compatible, de préférence souple, au moyen d'une couche uniforme (3) d'un matériau synthétique bio-compatible micro-poreux, épousant étroitement le noyau sphérique (36) ; la couche (3) est par exemple formée de deux bandes identiques (6, 7) de ce matériau synthétique bio-compatible micro-poreux, disposées suivant des plans moyens respectifs (18, 19) équatoriaux et mutuellement perpendiculaires, et mutuellement jointives bord à bord.

Utilisés après énucléation ou éviscération, de tels implants autorisent une colonisation par les tissus naturels, évitant ainsi les risques de rejet.

FIG. 2

EP 0 263 012 A1

# Description

## PROCEDE DE REALISATION D'UN IMPLANT INTRA-ORBITAIRE ET IMPLANT INTRA-ORBITAIRE.

La présente invention concerne la réalisation d'un implant intra-orbitaire destiné à être logé dans une cavité orbitaire après énucléation ou éviscération et à être raccordé à des muscles oculo-moteurs.

On a déjà proposé divers types de tels implants, dans le souci d'une reconstitution non seulement anatomique mais également, dans une certaine mesure, fonctionnelle après énucléation ou éviscération, afin d'obtenir un résultat esthétique le plus satisfaisant possible après que l'on ait adapté sur l'implant une prothèse reconstituant l'aspect du segment antérieur de l'oeil énucléé.

Ainsi, le brevet américain No 2 667 645 décrit la réalisation d'un implant à partir d'un noyau sphérique en matériau synthétique bio-compatible, sur une partie duquel on fixe un corset en grillage métallique permettant d'y ancrer des agrafes de fixation de certains des muscles moteurs de l'oeil énucléé ; cet implant présente l'inconvénient d'un manque d'uniformité et de sphéricité nuisant à sa mobilité dans la cavité orbitaire et créant même un risque de traumatisme et d'infection de la cavité orbitaire ; en outre, l'ancrage des muscles oculo-moteurs sur un tel implant est purement mécanique, ce qui implique un risque important de rejet de l'implant.

On retrouve ces inconvénients sur la plupart des implants intra-orbitaires destinés à être raccordés à des muscles oculo-moteurs qui sont actuellement connus.

Le but de la présente invention est de proposer la réalisation, à partir d'un noyau sphérique en matériau synthétique bio-compatible, d'un implant intra-orbitaire remédiant à ces inconvénients.

A cet effet, la présente invention propose un procédé de réalisation caractérisé en ce que l'on revêt intégralement le noyau sphérique d'une couche sensiblement uniforme d'un matériau synthétique bio-compatible micro-poreux, épousant étroitement le noyau sphérique.

La couche sensiblement uniforme, c'est-à-dire d'épaisseur sensiblement constante et d'aspect uni, dont est ainsi revêtu intégralement le noyau sphérique permet de conserver à celui-ci sa sphéricité ; le choix, pour la réalisation de cette couche sensiblement uniforme, d'un matériau synthétique bio-compatible micro-poreux, avantageusement constitué par du polytétrafluoréthylène micro-poreux du type décrit dans le brevet américain No3 953 566 et diffusé sous la marque commerciale "GORE-TEX" ou sous la marque commerciale "IMPRA", facilite l'ancrage des muscles, dans une application à l'énucléation, ou de la sclère, dans une application à l'éviscération, comme il apparaîtra plus loin, en autorisant une colonisation par les fibroblastes et par les vaisseaux dans toute l'épaisseur de la couche de revêtement du noyau sphérique, transformant progressivement cette couche en une véritable coque tissulaire et vasculaire limitant au maximum les risques d'expulsion de l'implant.

De préférence, on solidarise ladite couche avec le noyau sphérique, par exemple par collage, ou encore par suture.

Le matériau synthétique bio-compatible du noyau sphérique est de préférence souple et par exemple constitué par un élastomère choisi dans le groupe comportant les élastomères de silicone.

Le matériau synthétique bio-compatible micro-poreux est également de préférence souple, comme l'est par exemple le polytétrafluoréthylène micro-poreux du type indiqué plus haut, auquel cas on peut réaliser de façon particulièrement simple ladite couche, à partir d'un noyau de circonférence déterminée, par la succession des étapes consistant à :

a) réaliser deux bandes identiques plates, uniformes, du matériau synthétique souple, bio-compatible, micro-poreux, dont chacune présente un plan longitudinal de symétrie, deux bords longitudinaux de longueur approximativement égale à la moitié de ladite circonférence déterminée, mutuellement espacés d'une largeur approximativement égale au quart de ladite circonférence déterminée et raccordés mutuellement par deux bords transversaux hémicirculaires convexes présentant un diamètre approximativement égal au quart de ladite circonférence déterminée,

b) placer lesdites bandes respectivement de part et d'autre du noyau sphérique, dans une position dans laquelle leurs plans de symétrie sont équatoriaux et mutuellement perpendiculaires,

c) rabattre lesdites bandes autour du noyau sphérique jusqu'à une conformation dans laquelle elles sont mutuellement jointives bord à bord et se complètent mutuellement pour envelopper intégralement le noyau sphérique,

d) rendre permanente ladite conformation desdites bandes autour du noyau sphérique.

On peut réaliser l'étape d) en collant lesdites bandes au noyau sphérique, ou encore en suturant mutuellement, bord à bord, lesdites bandes auquel cas on les suture en outre de préférence également au noyau sphérique.

La présente invention ne se limite pas à un procédé de réalisation d'un implant intra-orbitaire mais, dans la mesure où cet implant présente une structure elle-même originale et avantageuse, elle s'étend également à des implants présentant cette structure, caractérisée en ce que le noyau sphérique est intégralement revêtu de ladite couche sensiblement uniforme de matériau synthétique bio-compatible micro-poreux, épousant étroitement le noyau sphérique et formée par exemple de deux bandes identiques dudit matériau synthétique bio-compatible micro-poreux, qui présentent des plans de symétrie respectifs équatoriaux et mutuellement perpendiculaires et sont mutuellement jointives bord à bord, quelle que soit la façon dont puissent être réalisés de tels implants.

L'invention sera mieux comprise si l'on se réfère à la description ci-dessous, relative à un mode de mise en oeuvre non limitatif, ainsi qu'aux dessins annexés qui font partie intégrante de cette description.

- La figure 1 montre une vue d'un implant conforme à la présente invention.

- La figure 2 montre une vue d'un tel implant en coupe par un plan équatorial.

- La figure 3 montre une vue en plan d'une feuille d'un matériau synthétique bio-compatible micro-poreux susceptible d'être utilisé pour la réalisation de la couche de revêtement du noyau.

- Les figures 4 et 5 montrent des vues en plan de deux bandes prélevées dans cette feuille en vue de la réalisation de ladite couche.

- La figure 6 illustre la mise en place de ces bandes autour du noyau, en une vue en perspective.

- La figure 7 montre une vue partielle, en coupe, analogue à celle de la figure 2, illustrant une phase initiale de la solidarisation des deux bandes avec le noyau.

- La figure 8 montre une vue en perspective, analogue à celle de la figure 1, illustrant cette phase initiale de solidarisation.

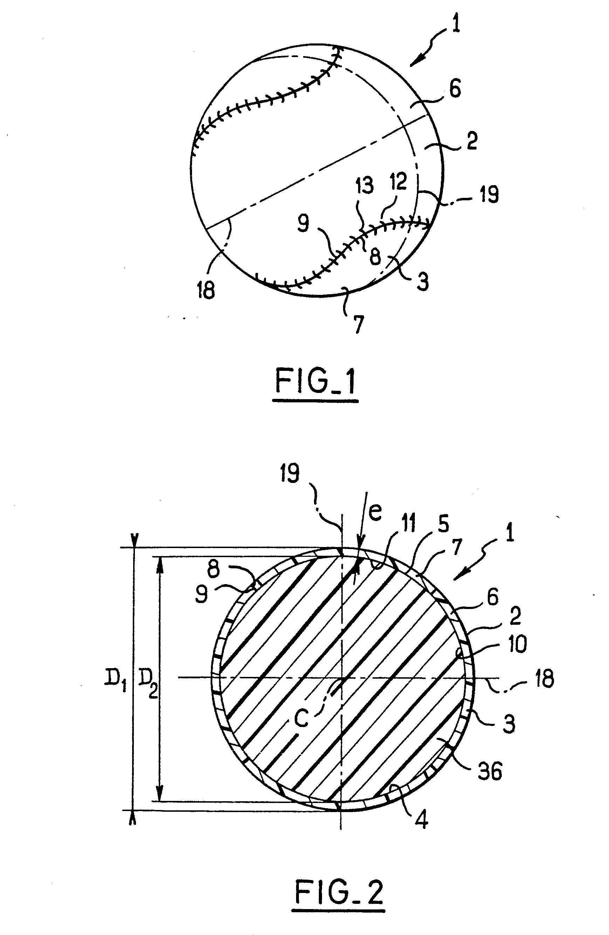

On se réfèrera en premier lieu aux figures 1 et 2, où l'on a illustré un implant 1 conforme à la présente invention.

Cet implant 1 présente une face extérieure 2 sensiblement sphérique de diamètre D1, déterminé empiriquement par un chirurgien en fonction des dimensions du globe occulaire énucléé ou éviscéré.

Cette face 2 est définie par une face extérieure d'une couche 3 d'épaisseur e sensiblement constante présentant par ailleurs une face intérieure 4 sphérique, de diamètre D2 égal à D1 diminué de 2 fois e, en contact étroit avec une face extérieure 5, également sphérique et de diamètre D2, d'un noyau plein 36 qui est ainsi intégralement couvert par la couche 3.

Le noyau 36 est en un matériau synthétique bio-compatible, de préférence souple, comme par exemple un élastomère de silicone ; on a obtenu de bons résultats aux essais avec un élastomère de silicone commercialisé sous la référence Q74535 par la Société DOW-CORNING catalysé avec 2,4 Dichlorobenzoyl, choisi pour sa bonne tolérance par l'organisme, mais on ne sortirait pas du cadre de la présente invention en choisissant d'autres matériaux synthétiques bio-compatibles pour la réalisation du noyau 36.

La couche 3, de préférence fixée par exemple par collage et/ou suture sur le noyau 36, est quant à elle réalisée en un matériau synthétique bio-compatible micro-poreux, par exemple du type diffusé sous la marque enregistrée GORE-TEX sous la référence "GORE-TEX E-PTFE SOFT TISSUE PATCH" et dont un procédé de réalisation est décrit dans le brevet américain No 3 953 566, avec une épaisseur e de l'ordre de 1 ou 2 mm, ou encore du type diffusé par la Firme IMPRA, avec une épaisseur e de 0,75 mm.

Bien que l'on ne sorte pas du cadre de la présente invention en réalisant la couche 3 en une seule pièce, cette couche 3 est constituée dans l'exemple illustré par deux bandes identiques 6, 7 dudit matériau synthétique bio-compatible micro-poreux, qui présentent des plans de symétrie respectifs 18, 19 équatoriaux en référence à la face sphérique 5 du noyau 36, c'est-à-dire passant par le centre géométrique C de cette face sphérique 5, lesquels plans de symétrie 18 et 19 sont en outre mutuellement perpendiculaires ; les deux bandes 6 et 7 présentant des bords périphériques respectifs 8, 9 communiquant à ces deux bandes 6, 7 des contours identiques, tels qu'elles soient jointives deux à deux par leurs bords 8, 9, et plus précisément sur la totalité de ces bords 8 et 9 pour assurer la couverture complète de la face 5 du noyau 6 sans surépaisseur dans la couche 3.

Les deux bandes 6 et 7 sont solidarisées mutuellement, et de préférence avec le noyau 36 par tout moyen approprié ; par exemple, si l'on désigne par 10 et 11 des faces respectives des bandes 6 et 7 accolées à la face 5 du noyau 36, c'est-à-dire définissant ensemble la face 4 de la couche 3, ces faces 10 et 11 des bandes 6 et 7 peuvent être collées, sur la totalité de leur surface, sur la face 5 du noyau 36 par une colle convenablement choisie ; on a obtenu de bons résultats aux essais en utilisant la colle référencée Q72947 de la Société DOW-CORNING, dans le cas d'un noyau 36 en élastomère de silicone du type indiqué plus haut et de bandes 6, 7 de couche 3 en polytétrafluoréthylène micro-poreux de l'un quelconque des types également indiqués plus haut, mais cet exemple n'est nullement limitatif ; les deux bandes 6 et 7 peuvent également être assemblées bord à bord par un surjet 12 en fil non résorbable 13, par exemple de polyester ; de préférence, comme il apparaîtra ultérieurement, ce surjet 12 comporte de place en place, de façon également répartie le long des bords 8 et 9, des points perforants, profonds 14 incluant non seulement le matériau des bandes 6 et 7 mais également le matériau du noyau 36, dans une zone localisée à proximité de la face 5 de celui-ci.

Les bandes 6 et 7 peuvent présenter de façon propre, résultant d'un mode de fabrication approprié, une conformation sphérique de leurs faces 10 et 11, telle que ces faces 10 et 11 s'adaptent exactement à la face 5 du noyau sphérique 36.

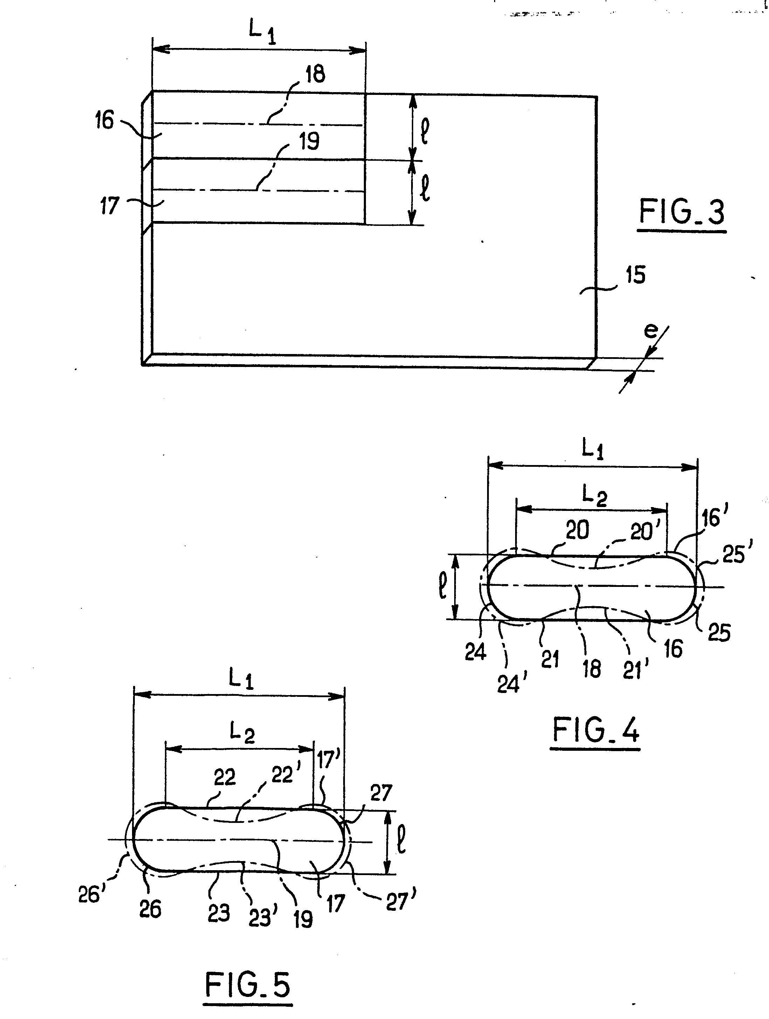

Cependant, dès lors que le matériau synthétique bio-compatible micro-poreux choisi est souple, on peut également fabriquer les bandes 6 et 7 de façon simple, économique, et néanmoins avec une approximation satisfaisante, par découpage, à plat, dans une feuille de ce matériau, présentant l'épaisseur e que l'on désire communiquer à la couche 3, comme on va le décrire à présent en référence aux figures 3 à 8.

A cet effet, comme le montre la figure 3, on commence par découper dans la feuille 15 deux bandes plates identiques 16, 17, présentant l'épaisseur e et une forme en plan rectangulaire, de longueur $L_1$ et de largeur l, avec :

$l = 1/4\pi\ D_2$ (où $\pi\ D_2$ correspond à la circonférence du noyau 36) $L_1 = 3l$.

Chacune des bandes 16 et 17 présente suivant sa plus grande dimension en plan $L_1$, perpendiculairement à sa plus petite dimension en plan l, un plan longitudinal de symétrie qui définira respectivement le plan de symétrie 18 de la bande 6 ou le plan de symétrie 19 de la bande 7 et porte de ce fait la référence 18 en ce qui concerne la bande 16 ou la référence 19 en ce qui concerne la bande 17.

Ensuite, comme le montrent les figures 4 et 5, les deux bandes 16 et 17 sont arrondies de façon identique, de façon à présenter deux bords longitudinaux rectilignes, mutuellement parallèles, de longueur $L_2$, égale à 21, mutuellement espacés de la largeur l, à savoir les bords 20 et 21 de la bande 16 et les bords 22 et 23 de la bande 17, et deux bords transversaux, hémicirculaires, convexes, de diamètre 1, centrés sur le plan longitudinal de symétrie respectivement correspondant 18, 19 et raccordant mutuellement les deux bords rectilignes précités, à savoir des bords 24 et 25 en ce qui concerne la bande 16 et des bords 26 et 27 en ce qui concerne la bande 17.

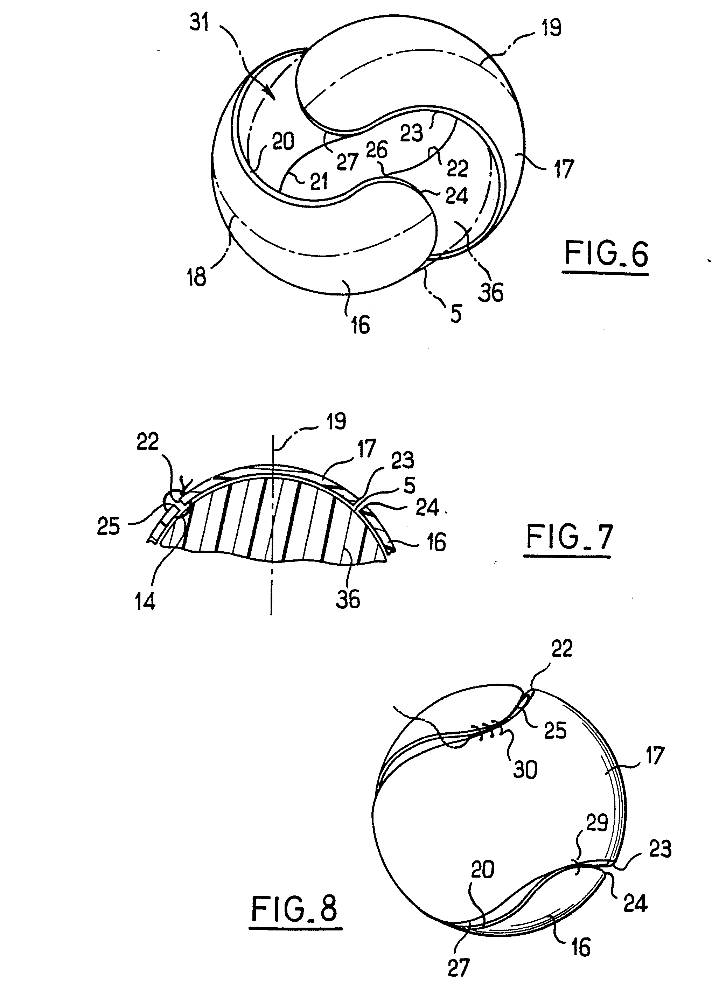

Ensuite, pour réaliser l'implant 1 et comme le montre la figure 6 où l'on a figuré en trait mixte le noyau 6 et sa face 5, on place les bandes 16 et 17 respectivement de part et d'autre du noyau 6, en positionnant leurs plans de symétrie 18 et 19 perpendiculairement l'un à l'autre et de telle sorte qu'ils passent par le centre (non représenté sur cette figure) du noyau 36.

Alors, en conservant cette position des plans 18 et 19, on déforme les bandes 16 et 17, à partir de leur conformation initiale plate, en les incurvant suivant leurs plans de symétrie 18 et 19 ainsi que transversalement par rapport à ces plans jusqu'à placer les bords 26 et 27 de la bande 17 au contact des bords 21 et 20, respectivement, de la bande 16 et les bords 24 et 25 de cette bande 16 au contact des bords 23 et 22 de la bande 17, respectivement, comme il ressort de l'examen des figures 6 à 8.

Les bords ainsi destinés à venir en contact mutuel se juxtaposent d'abord dans des zones immédiatement adjacentes aux plans 18 et 19 et, lorsque les bandes 16 et 17 sont destinées à être assemblées par suture pour constituer les bandes 6 et 7 de la couche 3, on réalise un maintien des bandes 16 et 17 entre elles en ces zones de juxtaposition initiale, par la pose de quelques points de suture tels que 29, 30, au fil non résorbable par exemple de polyester, par exemple 5/0, comme le montre la figure 8 ; ensuite, un surjet avec ce même fil permet de terminer la jonction bord à bord des deux bandes 16 et 17, qui se moulent progressivement sur la face 5 du noyau 36 de façon à l'épouser au mieux et à constituer les deux bandes 6 et 7 de la couche 3, se complétant pour assurer le recouvrement total de la face 5 du noyau 36.

Comme on l'a dit plus haut et comme le montre la figure 7, qui illustre le surjet en cours de réalisation, ce surjet concerne non seulement les bandes 16 et 17, mais également le noyau 36, du fait de la réalisation de points perforants, profonds 14 qui, en assurant la solidarisation des bandes 16 et 17, ou 6 et 7, avec le noyau 36, permettent de faire participer celui-ci à la résistance mécanique d'ensemble de l'implant 1.

En complément ou en remplacement de la liaison des bandes 16 et 17 par suture pour constituer les bandes 6 et 7 de la couche 3 de recouvrement du noyau 36, on peut également prévoir une fixation des bandes 16 et 17 par collage localement ou de préférence sur toute la surface de la face 5 du noyau 36 ; à cet effet, comme on l'a schématisé par une flèche 31 à la figure 6, on enduit localement ou, de préférence, intégralement la face 5 du noyau 36 d'une colle appropriée, par exemple du type indiqué plus haut, avant de rabattre sur cette face 5 les bandes 16 et 17 disposées de telle sorte que leurs plans moyens 18 et 19 soient équatoriaux, en référence au noyau 36, et perpendiculaires deux à deux ; en variante, on pourrait également enduire de colle ces bandes 16 et 17 elles-mêmes ; on rabat ensuite les bandes 16 et 17 comme on l'a dit plus haut, sur la face 5 du noyau 36, pour assurer leur juxtaposition bord à bord.

On obtient ainsi l'implant 1 illustré aux figures 1 et 2, dont les bandes 6 et 7 de la couche 3 sont constituées par les bandes 16 et 17, respectivement.

On remarquera que la forme des bandes 16 et 17 décrite en référence aux figures 3 à 5 implique une possibilité de déformation plastique de ces dernières pour permettre de les juxtaposer bord à bord en les moulant autour du noyau sphérique 36 ; le polytétrafluoréthylène micro-poreux du type indiqué plus haut offre une telle possibilité ; l'expérience a montré que les déformations plastiques subies par les bandes 16 et 17 lors de leur mise en place sur le noyau sphérique 36 sont suffisamment faibles pour n'entraîner que des variations négligeables dans l'épaisseur des bandes 16 et 17, c'est-à-dire pour n'entraîner qu'une asphéricité négligeable de la face 2 de la couche 3 de l'implant 1 obtenu.

Toutefois, lorsque le matériau constitutif des bandes 16 et 17 ne présente pas une aptitude suffisante à la déformation plastique ou lorsque, alors qu'il présente une telle aptitude suffisante, on désire que la face 2 de la couche 3 soit parfaitement sphérique, on peut découper les bandes 16 et 17 non pas selon la forme en plan décrite en référence aux figures 3 à 4 et illustrée en trait plein sur ces figures, mais selon une forme en plan, connue dans d'autres domaines de la technique et illustrée en trait mixte, respectivement en 16′ à la figure 4 et en 17′ à la figure 5 ; cette forme en plan, symétrique par rapport au plan 18 en ce qui concerne la bande 16′ et par rapport au plan 19 en ce qui concerne la bande 17′, se caractérise par deux bords transversaux identiques convexes, respectivement 24′, 25′ et 26′, 27′ approximativement en forme d'arcs de cercle centrés respectivement sur le plan 18 et sur le plan 19 avec un diamètre légèrement supérieur à l et un développement angulaire légèrement supérieur à 180°, ces deux bords transversaux étant raccordés mutuellement par deux bords longitudinaux identiques, curvilignes, concaves, respectivement 20′, 21′ et 22′, 23′, de longueur approximativement égale à $L_2$, définissant entre eux un étranglement de dimension transversale minimale légèrement inférieure à l ; longitudinalement, chaque bande 16′, 17′ présente une dimension légèrement supérieure à $L_1$ et correspondant à la différence entre la circonférence de la face 5 du noyau sphérique 6 et ladite dimension transversale minimale ; la mise en place et la fixation des bandes 16′ et 17′ autour du noyau

sphérique 36 s'effectue de la façon décrite à propos des bandes 16 et 17, pour obtenir un implant 1 du type illustré aux figures 1 et 2, dont les bandes 16′ et 17′ constituent respectivement les bandes 6 et 7.

L'implant 1 ainsi obtenu est ensuite stérilisé, par exemple à l'oxyde d'éthylène puis, après un temps de dégazage de l'ordre de trois semaines, peut être implanté soit après une énucléation, soit après une éviscération.

Dans le cas d'une application à l'énucléation, on veille, lors de cette énucléation, à préserver au maximum la conjonctive pour garder une profondeur suffisante des fornix supérieur et inférieur ; après dissection de la Tenon, chaque muscle droit, destiné à être réinséré sur l'implant, est isolé de la sclère et chargé par un fil non résorbable, par exemple en polyester, par exemple 5/0 ; on sectionne ensuite les muscles obliques qui peuvent éventuellement être également réinsérés sur l'implant et, dans ce cas, sont également chargés par un fil non résorbable ; ensuite, on sectionne le nerf optique et on pratique l'énucléation ; l'hémostase doit être parfaite, de même que la désinfection du champ opératoire ; alors, par exemple en utilisant un implant factice, stérile, de calibrage, on apprécie la quantité et la qualité de la Tenon et de la conjonctive et l'on évalue le diamètre D1 de l'implant 1 à utiliser, en fonction des dimensions de la cavité orbitaire ; le diamètre D1 varie généralement entre 14 mm et 20 mm, environ ; on met alors l'implant 1 en place, en suturant directement sur lui, c'est-à-dire sur la couche 3 et de préférence également, à travers celle-ci, sur le noyau 36 au moyen de points perforants profonds du type illustré en 14 à la figure 7, les muscles préalablement chargés, à savoir au moins les quatre muscles droits et de préférence également les muscles obliques ; on constatera ultérieurement une invasion vasculaire et fibroblastique du matériau de la couche 3, une véritable réinsertion musculaire se faisant progressivement, ce qui renforce l'ancrage des muscles sur l'implant 1 ; enfin, on ferme la Tenon et la conjonctive par points séparés, au fil résorbable 5/0, en veillant à ne pas mettre les sutures en tension ; un pansement au sérum glacé semi-compressif maintenu par une bande circonférentielle est préférable pendant les 24 premières heures suivant l'intervention ; un traitement antibiotique/ou anti-inflammatoire local ou général pendant 10 jours permet une réduction rapide de l'oedème post-opératoire.

Dans le cas d'une application à l'éviscération, on conserve la sclère et les muscles en place sur celle-ci, et l'on insère dans la sclère ainsi conservée et incisée latéralement l'implant 1, de taille choisie générale ment supérieure à celle des billes habituellement utilisées dans une telle application ; on laisse un dôme de la couche 3 non couvert par la sclère, dans une zone circulaire correspondant à une zone antérieure de l'oeil énucléé, pour conserver à la sclère sa forme naturelle, et on suture la sclère sur l'implant, par la couche 3 de celui-ci et de préférence également par son noyau 36 à travers la couche 3, par un surjet ou des points séparés auto-enfouis au moyen d'un fil non résorbable par exemple en polyester, par exemple 5/0, autour de ce dôme de la couche 3 non couvert par la sclère ; on peut également pratiquer à ce niveau une suture au fil résorbable, et réaliser par exemple deux points de suture au fil non résorbable entre l'implant 1 et la sclère dans des zones profondes de celle-ci, au niveau de l'extrémité des incisions latérales ; on referme ensuite la Tenon et la conjonctive, sur le dôme de la couche 3 ainsi découvert, comme on l'a indiqué plus haut à propos de l'énucléation, et en prenant les mêmes précautions lors de la suture ; le dôme de la couche 3 ainsi non recouvert de sclère sera ensuite colonisé par les tissus de recouvrement, à savoir la Tenon et la conjonctive et, dans le cas de l'utilisation d'un fil résorbable de surjet autour de ce dôme, cette colonisation prendra le relai du fil résorbable pour retenir la sclère sur l'implant.

Dans l'une et l'autre de ces applications, environ trois à quatre semaines après la pose de l'implant, le patient peut être adressé à un occulariste-prothésiste qui, de façon traditionnelle, réalise et adapte sur la conjonctive une prothèse reconstituant l'aspect extérieur de l'oeil énucléé ; par un moulage soigné de la prothèse, on obtient une mobilité de celle-ci à l'unisson avec l'implant.

## Revendications

1. Procédé de réalisation d'un implant intra-orbitaire, destiné à être logé dans une cavité orbitaire après énucléation ou éviscération et à être raccordé à des muscles oculo-moteurs, à partir d'un noyau sphérique (36) en matériau synthétique bio-compatible, caractérisé en ce que l'on revêt intégralement le noyau sphérique (36) d'une couche sensiblement uniforme d'un matériau synthétique bio-compatible micro-poreux, épousant étroitement le noyau sphérique (36).

2. Procédé selon la revendication 1, caractérisé en ce que l'on solidarise ladite couche (3) avec le noyau sphérique (36).

3. Procédé selon la revendication 2, caractérisé en ce que l'on colle ladite couche (3) au noyau sphérique (36).

4. Procédé selon l'une quelconque des revendications 2 et 3, caractérisé en ce que l'on suture ladite couche (3) au noyau sphérique (36).

5. Procédé selon l'une quelconque des revendications 1 à 4, caractérisé en ce que ledit matériau synthétique bio-compatible du noyau sphérique (36) est souple.

6. Procédé selon la revendication 5, caractérisé en ce que ledit matériau synthétique bio-compatible du noyau sphérique (36) est un élastomère choisi dans le groupe comportant les élastomères de silicone.

7. Procédé selon l'une quelconque des revendications 1 à 6, caractérisé en ce que ledit matériau synthétique bio-compatible micro-poreux est souple.

8. Procédé selon la revendication 7, caractérisé en ce que ledit matériau synthétique bio-compatible micro-poreux est un polytétrafluoréthylène micro-poreux.

9. Procédé selon l'une quelconque des revendications 7 et 8, ledit noyau sphérique (36) présentant une circonférence déterminée, caractérisé en ce que l'on réalise ladite couche (3) par la succession des étapes consistant à :

a) réaliser deux bandes identiques (16, 17) plates, uniformes, dudit matériau synthétique bio-compatible, micro-poreux, dont chacune présente un plan longitudinal de symétrie (18, 19), deux bords longitudinaux (20 à 23), de longueur (L2) approximativement égale à la moitié de ladite circonférence déterminée, mutuellement espacés d'une largeur (I) approximativement égale au quart de ladite circonférence déterminée et raccordés mutuellement par deux bords transversaux ( 24 à 27) hémi-circulaires convexes présentant un diamètre approximativement égal au quart de ladite circonférence déterminée,

b) placer lesdites bandes (16, 17) respectivement de part et d'autre du noyau sphérique (36), dans une position dans laquelle leurs plans de symétrie (18, 19) sont équatoriaux et mutuellement perpendiculaires,

c) rabattre lesdites bandes (16, 17) autour du noyau sphérique (36) jusqu'à une conformation dans laquelle elles sont mutuellement jointives bord à bord et se complètent mutuellement pour envelopper intégralement le noyau sphérique (36),

d) rendre permanente ladite conformation desdites bandes (16, 17) autour du noyau sphérique (36).

10. Procédé selon la revendication 9, caractérisé en ce que l'on réalise l'étape d) en solidarisant lesdites bandes (16, 17) avec le noyau sphérique (36).

11. Procédé selon la revendication 10, caractérisé en ce que l'on réalise l'étape d) en collant lesdites bandes (16, 17) au noyau sphérique (36).

12. Procédé selon l'une quelconque des revendications 9 à 11, caractérisé en ce que l'on réalise l'étape d) en suturant mutuellement, bord à bord, lesdites bandes (16, 17).

13. Procédé selon la revendication 12 en combinaison avec l'une quelconque des revendications 10 et 11, caractérisé en ce que l'on réalise l'étape d) en suturant en outre lesdites bandes (16, 17) au noyau sphérique (36).

14. Implant intra-orbitaire, destiné à être logé dans un cavité orbitaire après énucléation ou éviscération et à être raccordé à des muscles oculo-moteurs et comportant à cet effet un noyau sphérique (36) en matériau synthétique bio-compatible, caractérisé en ce que le noyau sphérique (36) est intégralement revêtu d'une couche uniforme (3) d'un matériau synthétique bio-compatible micro-poreux, épousant étroitement le noyau sphérique.

15. Implant selon la revendication 14, caractérisé en ce que ladite couche (3) est solidaire du noyau sphérique (36).

16. Implant selon la revendication 15, caractérisé en ce que ladite couche (3) est collée au noyau sphérique (36).

17. Implant selon l'une quelconque des revendications 15 et 16, caractérisé en ce que ladite couche (3) est suturée au noyau sphérique (36).

18. Implant selon l'une quelconque des revendications 14 à 17, caractérisé en ce que ledit matériau synthétique bio-compatible du noyau sphérique (36) est souple.

19. Implant selon la revendication 18, caractérisé en ce que ledit matériau synthétique bio-compatible du noyau sphérique (36) est un élastomère choisi dans le groupe comportant les élastomères de silicone.

20. Implant selon l'une quelconque des revendications 14 à 18, caractérisé en ce que ledit matériau synthétique bio-compatible micro-poreux est souple.

21. Implant selon la revendication 20, caractérisé en ce que ledit matériau synthétique bio-compatible micro-poreux est un polytétrafluoréthylène micro-poreux.

22. Implant selon l'une quelconque des revendications 14 à 21, caractérisé en ce que ladite couche (3) est formée de deux bandes identiques (6, 7) dudit matériau synthétique bio-compatible micro-poreux, qui présentent des plans de symétrie respectifs (18, 19) équatoriaux et mutuellement perpendiculaires et sont mutuellement jointives bord à bord.

23. Implant selon la revendication 22, caractérisé en ce que lesdites bandes (6, 7) sont solidaires du noyau sphérique (36).

24. Implant selon la revendication 23, caractérisé en ce que lesdites bandes (6, 7) sont collées au noyau (36).

25. Implant selon l'une quelconque des revendications 22 à 24, caractérisé en ce que lesdites bandes (6, 7) sont mutuellement suturées bord à bord.

26. Implant selon la revendication 25 en combinaison avec l'une quelconque des revendications 23 et 24, caractérisé en ce que lesdites bandes (6, 7) sont en outre suturées au noyau (36).

FIG_1

FIG_2

Neu eingereicht / Newly filed
Nouveau déposé

$L_1$

18

16

$\ell$

19

$\ell$

17

15

e

## FIG. 3

$L_1$

$L_2$

20     20'

16'

25'

24     21     16     25

24'     18     21'

## FIG. 4

$L_1$

$L_2$

22     22'     17'

27

26'     $\ell$

26     23     23'     19     17     27'

## FIG. 5

0263012

FIG.6

FIG.7

FIG.8

Office européen des brevets

# RAPPORT DE RECHERCHE EUROPEENNE

Numero de la demande

EP 87 40 2062

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | US-A-2 667 645 (H.R. MOULTON) <br> * Figures 1,3; colonne 4, lignes 17-41; colonne 5, lignes 57-71 * <br> --- | 1 | A 61 F 2/14 |
| A | US-A-3 364 501 (W.F. STAFFORD) <br> * Figure 1 * <br> ----- | 1 | |

DOMAINES TECHNIQUES RECHERCHES (Int. Cl.4)

A 61 F

Le présent rapport a été établi pour toutes les revendications

| Lieu de la recherche | Date d'achèvement de la recherche | Examinateur |
|---|---|---|
| LA HAYE | 14-12-1987 | ARGENTINI A. |

EPO FORM 1503 03.82 (P0402)